# FASCICULE DE BREVET EUROPEEN

(11) **EP 2 537 601 B1**
(45) Date de publication et mention de la délivrance du brevet: **07.04.2021**
(21) Numéro de dépôt: 11171360.8
(22) Date de dépôt: 24.06.2011
(51) Int. Cl.: B08B 17/00, C12Q 1/04, C12Q 1/22, G01N 33/52

(54) **Trousse et procédé de détection de biofilms**
Set und Verfahren für den Nachweis von Biofilmen
Biofilm detection kit and method

(43) Date de publication de la demande: 26.12.2012
(73) Titulaire: Realco, 1348 Louvain-la-Neuve (BE)
(72) Inventeur: Boels, Gauthier, 1040 Bruxelles (BE); Blackman, Gordon, 1380 Lasnes (BE); Calabozo, Almudena, 1348 Louvain-La-Neuve (BE)
(74) Mandataire: Calysta NV

(56) Documents cités:
- EP-A1- 1 491 505
- EP-A1- 1 536 225
- EP-A1- 2 243 821
- FR-A1- 2 928 457
- JP-A- 2005 210 997
- US-A- 6 117 423
- US-A1- 2002 085 980
- US-A1- 2003 205 247
- BONNIE MARCHANT ET AL: "Developing Fingerprints in Blood: A Comparison of Several Chemical Techniques", JOURNAL OF FORENSIC IDENTIFICATION, , vol. 57, no. 1 1 January 2007 (2007-01-01), pages 76-93, XP009511210, ISSN: 0895-173X Retrieved from the Internet: URL:https://www.ncjrs.gov/App/Publications /abstract.aspx?ID=238743

## Description

La présente invention se rapporte à un procédé et à une trousse de détection de biofilms, compatibles avec l'industrie alimentaire.

L'hygiène prend une importance croissante dans l'industrie alimentaire, dans les hôpitaux, dans les installations de potabilisation et de désalinisation d'eau, dans le traitement des eaux de procédé, et en particulier dans les eaux utilisées dans les tours de refroidissement et dans les nécessités de la vie courante, par exemple les lentilles de contact. On observe fréquemment que, lors de la circulation d'eau ou de substances riches en matière nutritive sur un support, des microorganismes circulant librement dans l'eau ou dans la matière nutritive peuvent adhérer à la surface. Ces microorganismes peuvent alors développer une matrice extracellulaire adhésive composée de substances polymères.

Une communauté de microorganismes adhérés à une surface et englobée dans une telle matrice est appelée un biofilm. Généralement ces biofilms sont composés de bactéries et de polymères organiques produits par celles-ci. Les biofilms se développent aujourd'hui sur tous types de support tels que les bandes transporteuses d'aliments dans l'industrie agroalimentaire, sur les supports destinés à maintenir une substance nutritive ou en tout cas organique pour une étape quelconque comme par exemple les crochets de maintien de viande et analogue. Les biofilms se développent également sur les plans de travail de l'horéca, sur les installations de nettoyage telles que les égouttoirs, les robinets, crédences, lave-vaisselles, etc.

On observe également la présence de biofilms dans les installations médicales telles que les salles d'opérations et autres dispositifs médicaux où des phases liquides sont présentes (liquides corporels, phase aqueuse de nettoyage après utilisation, etc.).

Finalement, d'autres industries sont sujettes à la présence de biofilms, comme l'industrie papetière, l'industrie du traitement de boues et tout autre industrie dans laquelle des matières solides végétales ou organiques sont mises à digérer en présence d'eau ou d'une solution aqueuse analogue.

On observe malheureusement que cette matrice est très résistante, et peut constituer une barrière pour des agents qui agiraient contre les microorganismes. Les traitements classiques à base de soude et/ou comportant différents biocides n'agissent pas de manière suffisamment efficace car ils ne pénètrent pas le biofilm sur toute son épaisseur ou sont inhibés par certaines molécules composant cette matrice. Le traitement n'est alors que partiellement efficace sur la surface supérieure du biofilm. En outre, ce dernier peut également piéger d'autres microorganismes, pathogènes notamment, que celui qui s'est installé initialement. Dès lors, un simple nettoyage des installations n'est généralement pas suffisant et requiert un traitement plus spécifique des zones contaminées par les biofilms.

Malheureusement, les traitements spécifiques de biofilms sont plus contraignants qu'une simple étape de nettoyage conventionnelle et requiert donc que les zones contaminées soient aisément identifiées. Si dans certains cas, ladite matrice extracellulaire est aisément identifiée lorsqu'elle est fortement développée, dans d'autres cas, le biofilm se développe insidieusement dans les installations et sa présence n'est détectée que lors de l'analyse de la qualité du produit fini.

En effet, il faut savoir que dans le domaine de l'industrie alimentaire, les biofilms se forment de manière inévitable (au vu de la richesse du milieu environnant). Les biofilms présentent une activité de croissance cyclique comprenant une phase de croissance durant laquelle se produit l'accumulation des microorganismes et une phase de détachement durant laquelle des morceaux entiers de biofilms se détachent par érosion et sous l'effet de leur propre poids. Lorsqu'un industriel est confronté à ce phénomène, il doit en réalité arrêter sa chaîne de production et effectuer le cycle de nettoyage pour éliminer le biofilm. Or ceci représente de nombreuses heures de travail et de perte de rendement de l'installation. En outre, ce type de détection permet de savoir qu'un biofilm est présent quelque part, mais ne permet pas de le localiser précisément.

Par conséquent, en pratique, la production n'est pas arrêtée et lorsque le biofilm est en phase de rupture, les lots produits sont contaminés et écartés jusqu'à ce que le niveau de contamination par les microorganismes des aliments produits soient à nouveau acceptable au vu des normes en vigueur.

Il existe donc un besoin de pouvoir localiser précisément la présence de biofilms dans ce type d'installation particulièrement contraignante puisque les produits fabriqués sont destinés à être ingérés par des êtres vivants, mais également dans tout type d'industrie sujette à la possibilité de développement de biofilms et pour lesquelles ce développement représente une problématique (ex : traitement de l'eau, circuit de refroidissement, santé, alimentation animale,...) et ce, afin de pouvoir y remédier de manière ponctuelle à tout moment, ou par exemple entre chaque lot de production ou bien à chaque arrêt de la production. En effet, si les surfaces de dispositifs de production ont été correctement sanitisées et sont exemptes de biofilms au départ d'une production, il est clair que le développement des biofilms sera ralenti. Par contre, s'il reste dans les installations nettoyées avant production un résidu non visible de biofilm, ceci constitue une amorce pour le développement d'un biofilm pendant la phase de production de l'aliment qui conduira inévitablement au gaspillage d'une partie de la production, contaminée par ledit biofilm.

Il a donc été développé ces dernières années, des techniques de détection de biofilms.

Par exemple, le document JP 2005210997 divulgue une composition colorante pour détecter des biofilms dans l'industrie alimentaire contenant du rouge de riz (monascine) qui est vaporisée sur des surfaces susceptibles de contenir des biofilms.

Cette composition comprend 92 % d'eau, 4 % de colorant et 4 % d'éthanol. Bien que compatible avec l'industrie alimentaire cette composition requiert une forte teneur en colorant, est peu compatible avec un milieu alcalin (utilisé pour sanitiser les installations de manière conventionnelle) et présente une couleur difficilement observable (justifiant vraisemblablement la teneur élevée en colorant).

De plus, comme on peut le constater à la lecture des exemples comparatifs de la présente demande de brevet, certaines substances alimentaires sont détectées par la monascine alors qu'il n'y a pas de biofilm présent.

La détection de faux positifs est réellement problématique. Il n'est en effet ni rentable ni écologique d'entamer des traitements de surfaces contaminées soi-disant par des biofilms à l'aide de substances spécifiques alors qu'il n'y a en réalité aucun biofilm et qu'il s'agit simplement de résidus alimentaires. Il faut en effet comprendre que pour les industriels, une étape d'élimination de biofilms nécessite généralement la mise à l'arrêt des installations de production, ce qui représente un coût non négligeable, qui ne doit pas être effectuée si ce n'est pas nécessaire.

Le document EP 1491505 divulgue quant à lui un procédé de mesure de la formation de biofilm dans des systèmes aqueux. Ce procédé comprend une première étape de placement de coupons d'échantillons dans le système aqueux. Il faut alors attendre qu'un biofilm se développe avant de récupérer les coupons. Les coupons sont ensuite colorés à l'aide d'un colorant, rincés et analysés par photométrie ou par comparaison avec une carte d'étalonnage.

De ceci, il résulte déjà que le procédé de détection selon ce document présente plusieurs inconvénients, en particulier le fait qu'il révèle certaines incertitudes et qu'il est réellement long. En effet, le placement de coupons doit être effectué dans des zones dans lesquelles on suspecte la présence de biofilms (laissant supposer qu'on les a déjà détectées auparavant) et d'attendre le développement de celui-ci. Bien que les bactéries soient rapides de développement, il n'est en aucun cas exclu qu'un biofilm ne se développe pas sur le coupon alors que l'installation est couverte de biofilms ou que le temps pendant lequel les coupons sont placés dans l'installation n'est pas adéquat pour qu'un biofilm se développe sur le coupon. Les bactéries présentant toujours une affinité plus élevée pour la matrice extracellulaire déjà formée sur l'installation à traiter que pour la surface lisse du coupon d'échantillonnage.

De plus, selon l'enseignement de ce document, avant coloration, les coupons sont rincés à l'azide pour éliminer la croissance bactérienne. La solution de coloration peut-être une solution aqueuse de safranine, bleu de coomassie, violet de cristal, rouge de ruthénium ou l'erythionine. La solution de rinçage est une solution contenant de l'azide et les coupons sont enfin lavés au DMSO après séchage.

Comme on peut aisément le constater, ce procédé décrit ne permet pas d'être appliqué dans des procédés alimentaires, l'azide NaN3 est toxique et explosif, sa manipulation est dangereuse. Ceci a pour résultat qu'en pratique, ce procédé est difficilement applicable d'ailleurs dans tout type d'industrie. En plus le DMSO n'est également pas applicable en industrie alimentaire. Enfin, l'utilisation de coupons est requise, ce qui limite ce procédé à des applications en circuit fermé (les coupons doivent être placés pendant une période de temps allant jusque 5 mois).

Ce document ne divulgue en outre aucune trousse de détection, utilise des dispositifs encombrants et tous les exemples utilisent de la safranine et de l'azide. Aucune application de l'enseignement de ce document n'est possible en agro-alimentaire et la couleur rouge n'est pas adéquate. De plus les coupons sont inapplicables aux surfaces ouvertes (bandes transporteuse) à cause du temps de séjour élevé (allant jusqu'à 5 mois), les coupons étant nécessaires au vu de la toxicité des substances utilisées.

Il existe donc un besoin de procurer un procédé et une trousse de détection fiable, rapide et peu encombrante, ne nécessitant pas d'appareils de mesure difficiles à utiliser ou à transporter comme des spectromètres et dont la détection peut être appliquée dans tout type d'industrie comme par exemple dans l'industrie alimentaire.

L'invention a pour but de pallier les inconvénients de l'état de la technique en procurant un procédé et une trousse de détection de biofilms permettant de détecter sur tout type de surface la présence de biofilms, particulièrement polyvalent, c'est-à-dire utilisable pour tout type d'application, y compris dans l'industrie agroalimentaire où d'un point de vue sanitaire, les biofilms doivent être détectés rapidement et précisément et de manière efficace, c'est-à-dire où la détection de faux négatif et de faux positif est limitée voire inexistante.

Pour résoudre ce problème, il est prévu suivant l'invention, un procédé de détection de biofilms, comprenant les étapes de :
- vaporisation d'une solution de coloration de biofilms consistant en un colorant qui est du bleu de Coomassie en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, sur une surface susceptible d'être contaminée par un biofilm, ladite surface étant une surface ouverte d'une installation,
- coloration dudit biofilm par ladite solution de coloration pendant une période de temps prédéterminée de moins de 15 minutes,
- vaporisation d'une solution de nettoyage comprenant ladite phase de dilution,
- dilution de ladite solution de coloration excédentaire par ladite solution de nettoyage pendant une période de temps prédéterminée de moins de 15 minutes, et
- détection et localisation précise dudit biofilm par observation de zones résiduelles colorées au bleu de Coomassie correspondant à des biofilms colorés par ledit colorant,
- ledit procédé de détection des biofilms étant un procédé de détection et de localisation précise des biofilms en moins d'une heure et compatible avec l'industrie agroalimentaire.

Plus particulièrement, une trousse de détection et de localisation de biofilms pour la mise en oeuvre du procédé selon l'invention, comprend:
- une solution prête à vaporiser de coloration de biofilms contenant un colorant en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, dans laquelle ledit colorant est du bleu de Coomassie,
- une solution prête à vaporiser de nettoyage comprenant ladite phase de dilution,
- ladite trousse de détection et de localisation de biofilms étant compatible avec l'industrie agroalimentaire.

Comme on peut le constater, la phase de dilution du colorant est compatible avec tout type d'application, mais également avec l'industrie agroalimentaire, ce qui permet de l'appliquer dans tout type d'installations industrielles et le colorant utilisé est aisément disponible sur le marché et particulièrement visible. De plus, la combinaison de la solution de coloration selon l'invention et de la solution de nettoyage permet de réduire la détection de faux positifs et améliore la sélectivité de la détection par rapport aux détections existantes.

En effet, selon la présente invention, ce n'est pas la détection de bactéries que l'on souhaite car ce type de détection fournirait inévitablement des résultats de faux négatifs. En effet, lorsque les bactéries provoquent la formation d'un biofilm, elles développent une matrice extracellulaire adhésive composée de substances polymères. Les bactéries adhèrent alors à la surface et sont confinées dans la matrice polymère composée principalement de glycoprotéines. Ce sont donc ces glycoprotéines qu'il y a donc lieu de détecter et non les polysaccharides généralement utilisés dans l'art antérieur pour détecter les biofilms. La détection de polysaccharides peut quant à elle fournir quantité de résultats non spécifiques par la présence de faux positifs (les polysaccharides sont très présent dans les résidus de l'agroalimentaire) ou de faux négatifs. Si la surface est bien nettoyée et donc que peu de polysaccharides issus de résidus d'aliments sont présents, les polysaccharides de bactéries peuvent ne pas être détectés étant donné que les bactéries sont confinées dans cette matrice polymère et ne sont donc pas accessibles pour la coloration, surtout si le temps de pause du colorant est peu élevé.

De plus, généralement, ce sont des colorants à base de rouge qui sont utilisés, mais ceux-ci sont fort peu visibles, s'ils parviennent à atteindre les polysaccharides des bactéries confinées dans les biofilms, au travers de cette matrice constituant le biofilm.

C'est pour cette raison que la trousse selon l'invention présente une rapidité de détection et une spécificité très avantageuse car elle cible les glycoprotéines de la matrice extracellulaire du biofilm et réduit donc la présence de faux négatifs ou de faux positifs et cible les glycoprotéines très accessibles par le colorant et peu présentes dans les aliments ou autres résidus. Le biofilm est donc rapidement détecté et de manière sélective.

La trousse de détection selon l'invention comprend simplement une première solution de coloration à vaporiser et une solution de nettoyage à vaporiser ensuite dont les temps de pause sont courts (moins de 15 minutes). Ceci signifie que généralement, en moins d'une heure, de préférence en moins d'une demi-heure, les biofilms sont détectés et localisés précisément.

L'aspect pratique de la trousse selon l'invention est particulièrement avantageuse en ce qu'elle ne demande pas d'installation coûteuse pour détecter le biofilm, l'œil de l'utilisateur suffit, ce qui est dû au choix particulier du colorant, aisément identifiable à l'œil nu, différent des couleurs généralement trouvées dans tous les types d'industries traitant de la matière végétale ou organique (peu d'aliments bleus, au contraire du rouge). Une simple vaporisation de la première solution suivie de la deuxième suffit.

Avantageusement, la trousse de détection selon l'invention comprend en outre une composition de décoloration, compatible avec l'industrie agroalimentaire, qui permet ensuite de faire disparaître la couleur bleue de l'installation si nécessaire. En effet, dans certaines applications, les matériaux utilisés sont poreux, comme les caoutchoucs des bandes transporteuses. Afin de ne pas maintenir de couleur bleue sur ces matières spécifiques, une solution de décoloration sera utilisée. Il n'est en effet pas avantageux que la couleur bleue persiste après utilisation car ceci pourrait nuire aux étapes de détection ultérieures. La couleur bleue du biofilm étant bien entendu éliminée avec le biofilm.

Dans une forme de réalisation particulière, ladite composition de décoloration est une phase solide d'un agent oxydant qui peut donc simplement être répandue sur la zone traitée, de préférence préalablement mouillées pour favoriser l'activation de l'agent oxydant dans l'eau. La présence de la composition de décoloration sous forme d'une phase solide est avantageuse pour la conservation du caractère oxydant de la composition de décoloration au cours du temps.

Dans une variante selon l'invention, ladite composition de décoloration est une solution aqueuse d'un agent oxydant, ce qui lui permet d'être aisément appliquée sur la zone à traiter par simple vaporisation, surtout lorsqu'elle est verticale.

Dans une forme particulière de la trousse de détection de biofilms selon l'invention, ledit agent oxydant est choisi dans le groupe constitué du percarbonate de soude, de l'hypochlorite de sodium, de l'eau oxygénée, les perborates, les persulfates ou encore les peroxydes ou et leurs mélanges et leurs dérivés comme par exemple le percarbamate d'urée.

En particulier, ladite phase de dilution comprend de 40 à 50 %, de manière plus préférentielle environ 45 % en volume d'éthanol, en particulier d'éthanol absolu, par rapport au volume final de ladite phase de dilution, de 8 à 12 %, plus particulièrement environ 10% en volume d'acide acétique, en particulier d'acide acétique glacial, par rapport au volume final de ladite phase de dilution, et de 40 à 50 %, de manière plus préférentielle environ 45 % en volume d'eau, par rapport au volume final de ladite phase de dilution.

Comme on peut le constater aisément, cette phase de dilution ne comprend que des substances ingérables et compatibles avec l'industrie agroalimentaire, que l'on peut aisément se procurer et peu coûteuses.

D'autres formes de réalisation de la trousse de détection suivant l'invention sont indiquées dans les revendications annexées.

Comme mentionné précédemment, le procédé selon la présente invention est rapide, peu coûteux en terme de temps et d'équipement à utiliser, efficace par sa sélectivité et sa rapidité de détection et compatible avec l'industrie agroalimentaire, donc polyvalent pour tout type d'industrie.

Avantageusement, une ou plusieurs des étapes choisies dans le groupe de l'étape de vaporisation de ladite solution de coloration, de vaporisation de ladite solution de nettoyage et de détection dudit biofilm est précédée d'une étape de rinçage à l'eau, ce qui permet d'éliminer les surplus de colorant, sur la zone à traiter ou diluée dans la solution de nettoyage, ce qui permet d'améliorer la sélectivité du procédé de détection de biofilms suivant l'invention.

Dans un mode de réalisation préférentiel selon la présente invention, le procédé comprend en outre une étape de décoloration desdites zones résiduelles colorées au bleu de coomassie par application d'une composition de décoloration.

Avantageusement, ladite étape de décoloration est effectuée par application d'une composition de décoloration solide, en particulier pulvérulente d'un agent oxydant, sur la surface à traiter préalablement mouillée pour favoriser l'activation de l'agent oxydant.

Dans une variante selon l'invention, ladite étape de décoloration est effectuée par application d'une composition de décoloration liquide d'un agent oxydant.

De préférence, selon l'invention, ladite période de temps prédéterminée est comprise entre 3 et 15 minutes, de préférence entre 4 et 10 minutes et est généralement d'environ 5 minutes. Ceci permet bien entendu de rapidement détecter le biofilm, mais reflète également l'efficacité du procédé selon l'invention qui peut être mis en œuvre rapidement et sa sélectivité (la substance à détecter est réellement ciblée et vite détectée).

De manière plus préférentielle, ladite surface susceptible d'être contaminée par un biofilm est une surface ouverte d'une installation, en particulier d'une installation de l'industrie agroalimentaire. Il est en effet préférable que la surface sur laquelle un biofilm doit être détecté par le procédé selon l'invention soit une surface visible à l'œil, c'est ce que l'on appelle généralement une surface ouverte. Toutefois, le procédé selon l'invention peut également être appliqué sur des surfaces moins accessibles ou non visibles, mais elles devront sans doute être démontées en vue de l'étape de détection, par exemple, des tubulures peuvent être traitées, par exemple par circulation, mais elles devront vraisemblablement être ouverte pour détecter le résultat.

Bien entendu, bien que ce ne soit pas nécessaire selon la présente invention, le procédé peut également recourir à l'utilisation de coupons placés dans des installations fermées. Une fois récupérés, les coupons sont alors traités comme n'importe quelle surface ouverte au sens de la présente invention.

D'autres formes de réalisation du procédé suivant l'invention sont indiquées dans les revendications annexées.

La présente invention se rapporte également à une utilisation d'une solution de coloration de biofilms consistant en un colorant qui est du bleu de Coomassie en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, et d'une solution prête à vaporiser de nettoyage comprenant ladite phase de dilution, sur une surface susceptible d'être contaminée par un biofilm, ladite surface étant une surface ouverte, en particulier dans une installation de l'industrie agroalimentaire, afin de détecter et localiser ledit biofilm.

D'autres caractéristiques, détails et avantages de l'invention ressortiront de la description donnée ci-après, à titre non limitatif et en faisant référence aux exemples mentionnés.
La figure 1 est une vue en perspective d'une trousse de détection de biofilms selon l'invention.
La figure 2 est une photographie d'un élément de l'industrie agroalimentaire traité par le kit de détection de biofilms selon la présente invention qui montre la détection de biofilms.

Sur les figures, les éléments identiques ou analogues portent les mêmes références.

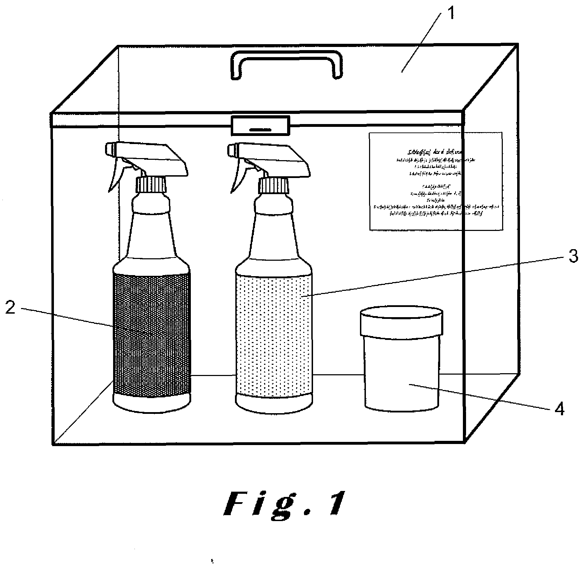

La figure 1 illustre la trousse de détection 1 de biofilm comprenant une solution de coloration 2 de biofilms contenant du bleu de coomassie en solution dans une phase de dilution compatible avec l'industrie agroalimentaire. Le colorant peut évidemment, en fonction des applications requises éventuellement comprendre un autre colorant en mélange, mais comportera de toutes façons, du bleu de coomassie dans le but de détecter des biofilms.

La phase de dilution de la solution de coloration 2 comprend, dans cette forme de réalisation avantageuse illustrée 45 % en volume d'éthanol absolu, 10 % en volume d'acide acétique glacial et 45 % en volume d'eau par rapport au volume final de ladite phase de dilution.

La trousse de détection 1 comprend en outre une solution de nettoyage 3 avantageusement constituée de ladite phase de dilution. Ceci permet bien entendu de diluer le colorant non lié aux protéines du biofilm.

La trousse de détection 1 de biofilms comprend en outre avantageusement une composition de décoloration 4, compatible avec l'industrie agroalimentaire. Dans la forme de réalisation illustrée, la composition de décoloration 4 est une phase solide d'un agent oxydant composé de percarbonate de soude. Dans une variante non illustrée selon l'invention, la composition de décoloration 4 est une solution aqueuse d'un agent oxydant, comme par exemple d'une solution d'hypochlorite de sodium ou d'eau oxygénée.

Dans une utilisation avantageuse de la trousse de détection 1 de biofilms, tout d'abord, la surface à traiter pour détecter si un biofilm est présent ou non, par exemple une surface ouverte de l'industrie agroalimentaire est rincée et ensuite vaporisée à l'aide de la solution de coloration 2. La coloration est laissé à agir pendant environ 5 minutes et la surface à traiter est ensuite rincée pour en éliminer la solution de coloration excédentaire. Généralement, la quantité de solution de coloration utilisée sera d'environ 6,5 µl/cm² de surface à traiter, soit 65,2 ml/m².

La surface à traiter est ensuite, selon les recommandations vaporisée à l'aide de la solution de nettoyage 3 que l'on laisse agir pendant environ 5 minutes. La solution de nettoyage est avantageusement constituée de la phase de dilution du colorant de la solution de coloration 2 de la trousse de détection de biofilm selon la présente invention. La solution de nettoyage permet donc d'éliminer les traces éventuelles de bleu de coomassie excédentaire qui n'adhère pas au biofilm par effet de dilution dans la phase de dilution. Généralement, la quantité de solution de coloration utilisée sera d'environ 21 ,7 µl/cm² de surface à traiter, soit 217 ml/m².

La surface est ensuite rincée à l'eau pour éliminer les traces de bleu de coomassie résiduelles dilué dans la solution de nettoyage 3 et la surface à traiter est ensuite laissée à reposer pendant 5 minutes. Lorsque de la coloration bleue persiste sur la surface à traiter, elle indique la présence d'un biofilm et permet donc sa détection rapide en environ moins de 20 minutes. Finalement, la composition de décoloration sous forme de réactif en poudre dans la trousse selon l'invention est répartie sur la surface à traiter humide pour éliminer la coloration bleue de la surface à traiter.

### EXEMPLE 1.- (exemple utile à la compréhension de l'invention, mais n'en constituant pas un mode de réalisation)

5 jeux de coupons différents ont été recouverts de différentes matières organiques utilisées fréquemment dans l'industrie agroalimentaire selon les protocoles mentionnés ci-dessous.
a) beurre : les coupons ont été enduits de beurre manuellement
b) amidon : des coupons sont placés dans une coupelle et recouverts d'un ml d'eau de cuisson de riz, riche en amidon. Les coupons sont ensuite laissés à sécher à l'air libre.
c) carboxyméthylcellulose : des coupons sont placés dans une coupelle et recouverts d'un ml d'une solution de carboxvméthylcellulose (CMC). Les coupons sont ensuite laissés à sécher à l'air libre. La concentration de carboxvméthylcellulose de la solution utilisée est de 500 mg de CMC/50 ml d'eau déminéralisée.
d) gélatine : des coupons sont placés dans une coupelle et recouverts d'un ml d'une solution de gélatine. Les coupons sont ensuite laissés à sécher à l'air libre. La concentration de la solution de gélatine est de 3,6 % de gélatine, obtenue par dissolution de 3,6 g de gélatine commerciale dans 100 ml d'eau chaude.
e) lait : des coupons sont placés dans une coupelle et 1 ml d'une solution de lait diluée 100 fois par de l'eau est versée sur chaque coupon. Les coupons sont ensuite laissé sécher à l'air libre.

Les coupons sont alors disposés dans la solution colorée durant 30 minutes contenant environ 0,25 g de bleu de Coomassie R250 dans une phase de dilution comprenant 45 ml d'éthanol pur, 45 ml d'eau distillée et 10 ml d'acide acétique glacial. Les coupons sont ensuite plongés dans 100 ml de solution de nettoyage constituée approximativement de la même phase de dilution. Ensuite, on a laissé les coupons à sécher à l'air libre.

L'analyse visuelle des coupons permet de constater que le beurre, la carboxyméthylcellulose, la gélatine et l'amidon ne sont pas colorés par le bleu de Coomassie. De légères traces subsistent avec le lait dilué 100 fois.

Comme on peut le constater, la trousse de détection de biofilm selon la présente invention permet de détecter les biofilms avec une grande spécificité.

### EXEMPLE 2.-exemple industriel dans une industrie aqroalimentaire.

Des installations d'un abattoir ont été, après nettoyage conventionnel testées pour la présence de biofilms à l'aide de la trousse selon l'invention. Les installations après nettoyage conventionnel présentaient un aspect visuel de propreté très bon. Les zones testées pour la détection de biofilms sont principalement les salles de plumaison, éviscération et bridage.

Sur les surfaces légèrement humides, la solution de coloration de la trousse selon l'invention a été vaporisée sur tous les dispositifs de ces salles telles que crochets, arrache-tête, lames, récipients inox, godets, etc. et laissée à agir pendant 5 minutes. La solution de coloration comprenant 0,1 g de bleu de coomassie dans 100 ml de phase de dilution comprenant 45 ml d'eau, 45 ml d'éthanol absolu et 10 ml d'acide acétique glacial.

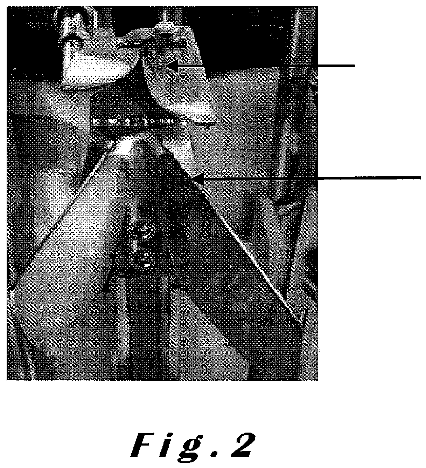

Les surfaces des éléments testés ont été rincées avec de l'eau pour éliminer le colorant en excès et ensuite vaporisées par la solution nettoyante comprenant ladite phase de dilution. La solution de nettoyage a été laissée à agir pendant 5 minutes et les éléments testés pour la détection de biofilms ont été rincés et frottés mécaniquement pour apporter une légère action mécanique et éliminer les colorations résiduelles et/ou faux positifs éventuels. Les résultats ont ensuite été observés. On peut se rendre compte très facilement que les problèmes de contamination rémanente sur certaines surfaces sont provoquées par la présence de biofilm, comme par exemple sur les éléments testés tels que les crochets de plumaison, les lames, les godets, récipients et les arraches têtes. Seuls les arraches tête sont illustrés à la figure 2 bien que les autres éléments soient également contaminés. Malgré la qualité du nettoyage effectuée par la société, certains appareils sont contaminés de manière parfois sévère par un biofilm. Ce biofilm est à l'origine de résultats microbiologiques non conformes

### EXEMPLE COMPARATIF 1.-

Les 5 jeux de coupons différents de l'exemple 1 selon l'invention ont été recouverts des mêmes différentes matières organiques utilisées fréquemment dans l'industrie agroalimentaire à l'exception du fait que pour le lait, deux tests ont été réalisés, la dilution du lait appliquée était de 00 fois comme dans l'exemple 1 , mais aussi de 20 fois.

Les coupons sont alors disposés dans la solution colorée contenant environ 3 g de rouge de riz (monascine) dans 100 ml d'une phase de dilution comprenant de l'eau et de l'éthanol dans un rapport 1/1 durant 15 minutes, puis pour le rinçage, 10 minutes dans l'eau distillée.

L'analyse visuelle des coupons permet de constater que le beurre, la carboxyméthylcellulose, ne sont pas colorés par le rouge de riz (monascine), par contre le lait (aux deux dilutions), l'amidon et la gélatine sont colorés par ce colorant, ce qui le rend non spécifique et rend son usage complexe. Il faut remarquer ici que le protocole du test selon le document antérieur JP2005210997 a été adapté pour pouvoir comparer les résultats avec ceux obtenus pour la trousse selon la présente invention.

### EXEMPLE COMPARATIF 2.-

Les 5 jeux de coupons différents de l'exemple 1 ont été recouverts des mêmes différentes matières organiques utilisées fréquemment dans l'industrie agroalimentaire à l'exception du fait que pour le lait, deux tests ont été réalisés, la dilution du lait appliquée était de 00 fois comme dans l'exemple 1, mais aussi de 20 fois.

Les coupons sont alors disposés dans la solution colorée contenant environ 36 g/l de safranine dans une phase de dilution comprenant de l'eau déminéralisée, de la méthyléthylcétone et de l'éthanol durant 15 minutes, puis pour le rinçage, 10 minutes dans de l'eau déminéralisée.

L'analyse visuelle des coupons permet de constater que le beurre, la carboxyméthylcellulose, et la gélatine ne sont pas colorés par la safranine, par contre le lait (aux deux dilutions), et l'amidon sont colorés par ce colorant, ce qui le rend non spécifique et rend son usage complexe. Il faut remarquer ici que le protocole du test selon le document antérieur EP1491505 a été adapté pour d'une part être applicable dans l'industrie agroalimentaire et pour pouvoir comparer les résultats avec ceux obtenus pour la trousse selon la présente invention.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé de détection de biofilms, comprenant les étapes de :
- vaporisation d'une solution de coloration de biofilms consistant en un colorant qui est du bleu de Coomassie en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, sur une surface susceptible d'être contaminée par un biofilm, ladite surface étant une surface ouverte d'une installation,
- coloration dudit biofilm par ladite solution de coloration pendant une période de temps prédéterminée de moins de 15 minutes,
- vaporisation d'une solution de nettoyage comprenant ladite phase de dilution,
- dilution de ladite solution de coloration excédentaire par ladite solution de nettoyage pendant une période de temps prédéterminée de moins de 15 minutes, et
- détection et localisation précise dudit biofilm par observation de zones résiduelles colorées au bleu de Coomassie correspondant à des biofilms colorés par ledit colorant,
- ledit procédé de détection des biofilms étant un procédé de détection et de localisation précise des biofilms en moins d'une heure et compatible avec l'industrie agroalimentaire.

2. Procédé de détection de biofilms selon la revendication 1, dans lequel une ou plusieurs des étapes choisies dans le groupe de l'étape de vaporisation de ladite solution de coloration, de vaporisation de ladite solution de nettoyage et de détection dudit biofilm est précédée d'une étape de rinçage à l'eau.

3. Procédé de détection de biofilms selon la revendication 1 ou la revendication 2, comprenant en outre une étape de décoloration desdites zones résiduelles colorées au bleu de Coomassie par application d'une composition de décoloration.

4. Procédé de détection de biofilms selon la revendication 3, dans lequel ladite étape de décoloration est effectuée par application d'une composition de décoloration solide, en particulier pulvérulente d'un agent oxydant.

5. Procédé de détection de biofilms selon la revendication 3, dans lequel ladite étape de décoloration est effectuée par application d'une composition de décoloration liquide d'un agent oxydant.

6. Procédé selon l'une quelconque des revendications 1 à 5, dans lequel ladite période de temps prédéterminée est comprise entre 3 et 15 minutes, de préférence entre 4 et 10 minutes et est généralement 5 minutes.

7. Procédé selon l'une quelconque des revendications 1 à 6, dans lequel ladite surface ouverte susceptible d'être contaminée par un biofilm est une surface d'une installation de l'industrie agroalimentaire, traitement de l'eau, circuit de refroidissement, santé et alimentation animale.

8. Trousse de détection (1) et de localisation de biofilms pour la mise en œuvre du procédé selon l'une quelconque des revendications 1 à 7, comprenant :
- une solution prête à vaporiser de coloration (2) de biofilms contenant un colorant en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, dans laquelle ledit colorant est du bleu de Coomassie,
- une solution prête à vaporiser de nettoyage (3) comprenant ladite phase de dilution
- ladite trousse de détection et de localisation de biofilms étant compatible avec l'industrie agroalimentaire.

9. Trousse de détection (1) de biofilms selon la revendication 8, comprenant en outre une composition de décoloration (4), compatible avec l'industrie agroalimentaire.

10. Trousse de détection (1) de biofilms selon la revendication 9, dans laquelle ladite composition de décoloration (4) est une solution aqueuse d'un agent oxydant.

11. Trousse de détection (1) de biofilms selon la revendication 9, dans laquelle ladite composition de décoloration (4) est une phase solide d'un agent oxydant.

12. Trousse de détection (1) de biofilms selon la revendication 10 ou 11, ledit agent oxydant étant choisi dans le groupe du percarbonate de soude, de l'hypochlorite de sodium, de l'eau oxygénée, les perborates, les persulfates ou encore les peroxydes ou de leur mélanges et dérivés comme par exemple le percarbamate d'urée.

13. Trousse de détection (1) de biofilms selon l'une quelconque des revendications 8 à 12, dans laquelle ladite phase de dilution comprend de 40 à 50 %, de manière plus préférentielle 45 % en volume d'éthanol, en particulier d'éthanol absolu, par rapport au volume final de ladite phase de dilution, de 8 à 12 %, plus particulièrement 10% en volume d'acide acétique, en particulier d'acide acétique glacial, par rapport au volume final de ladite phase de dilution, et de 40 à 50 %, de manière plus préférentielle 45 % en volume d'eau, par rapport au volume final de ladite phase de dilution.

14. Utilisation d'une solution prête à vaporiser de coloration de biofilms consistant en un colorant qui est du bleu de Coomassie en solution dans une phase de dilution compatible avec l'industrie agroalimentaire, comprenant de 35 à 55 % en volume d'éthanol, de 7 à 13 % en volume d'acide acétique et de 35 à 55 % en volume d'eau, par rapport au volume final de ladite phase de dilution, et d'une solution prête à vaporiser de nettoyage comprenant ladite phase de dilution, sur une surface susceptible d'être contaminée par un biofilm, ladite surface étant une surface ouverte, en particulier dans une installation de l'industrie agroalimentaire, afin de détecter et localiser ledit biofilm.

## Patentansprüche

1. Verfahren zum Nachweis von Biofilmen, umfassend die folgenden Schritte:
- Verdampfen einer Lösung zum Färben von Biofilmen, bestehend aus einem Farbstoff, der Coomassie-Brillantblau ist, in Lösung in einer mit der Lebensmittelindustrie verträglichen Verdünnungsp hase, umfassend 35 bis 55 Volumen-% Ethanol, 7 bis 13 Volumen-% Essigsäure und 35 bis 55 Volumen-% Wasser in Bezug auf das Endvolumen der Verdünnungsphase, auf einer Oberfläche, die möglicherweise durch einen Biofilm kontaminiert ist, wobei die Oberfläche eine offene Oberfläche einer Anlage ist,
- Färben des Biofilms mit der Färbelösung während einer vorbestimmten Zeitdauer von weniger als 15 Minuten,
- Verdampfen einer Reinigungslösung, die die Verdünnungsphase umfasst,
- Verdünnen der überschüssigen Färbelösung mit der Reinigungslösung während einer vorbestimmten Zeitdauer von weniger als 15 Minuten, und
- Nachweis und genaues Lokalisieren des Biofilms durch Beobachtung von mit Coomassie-Brillantblau gefärbten Restbereichen, die den mit dem Farbstoff gefärbten Biofilmen entsprechen,
- wobei das Verfahren zum Nachweis der Biofilme ein Verfahren zum Nachweis und zum genauen Lokalisieren der Biofilme in weniger als einer Stunde ist und das mit der Lebensmittelindustrie verträglich ist.

2. Verfahren zum Nachweis von Biofilmen nach Anspruch 1, wobei einem oder mehreren Schritten, die ausgewählt sind aus der Gruppe des Schritts des Verdampfens der Färbelösung, des Verdampfens der Reinigungslösung und des Nachweises des Biofilms, ein Schritt des Spülens mit Wasser vorausgeht.

3. Verfahren zum Nachweis von Biofilmen nach Anspruch 1 oder Anspruch 2, weiter umfassend einen Schritt des Entfärbens der mit Coomassie-Brillantblau gefärbten Restbereiche durch Auftragen einer Entfärbungszusammensetzung.

4. Verfahren zum Nachweis von Biofilmen nach Anspruch 3, wobei der Schritt des Entfärbens durch Auftragen einer festen Entfärbungszusammensetzung, insbesondere einer pulverförmigen eines Oxidationsmittels, durchgeführt wird.

5. Verfahren zum Nachweis von Biofilmen nach Anspruch 3, wobei der Schritt des Entfärbens durch Auftragen einer flüssigen Entfärbungszusammensetzung eines Oxidationsmittels durchgeführt wird.

6. Verfahren nach einem der Ansprüche 1 bis 5, wobei die vorbestimmte Zeitdauer zwischen 3und 15 Minuten, vorzugsweise zwischen 4 und 10 Minuten, umfasst ist, und im Allgemeinen 5 Minuten beträgt.

7. Verfahren nach einem der Ansprüche 1 bis 6, wobei die offene Oberfläche, die möglicherweise durch einen Biofilm kontaminiert ist, eine Oberfläche einer Lebensmittelindustrie-, Wasseraufbereitungs-, Kühlkreis-, Gesundheits- und Tierfutteranlage ist.

8. Set zum Nachweis (1) und zum Lokalisieren von Biofilmen zum Einsatz des Verfahrens nach einem der Ansprüche 1 bis 7, umfassend:
- eine verdampfungsbereite Lösung zum Färben (2) von Biofilmen, die einen Farbstoff in Lösung in einer mit der Lebensmittelindustrie verträglichen Verdünnungsphase enthält, umfassend 35 bis 55 Volumen-% Ethanol, 7 bis 13 Volumen-% Essigsäure und 35 bis 55 Volumen-% Wasser in Bezug auf das Endvolumen der Verdünnungsphase, wobei der Farbstoff Coomassie-Brillantblau ist,
- eine verdampfungsbereite Reinigungslösung (3), die die Verdünnungsphase umfasst,
- wobei das Set zum Nachweis und zum Lokalisieren von Biofilmen mit der Lebensmittelindustrie verträglich ist.

9. Set zum Nachweis (1) von Biofilmen nach Anspruch 8, weiter umfassend eine mit der Lebensmittelindustrie verträgliche Entfärbungszusammensetzung (4).

10. Set zum Nachweis (1) von Biofilmen nach Anspruch 9, wobei die Entfärbungszusammensetzung (4) eine wässrige Lösung eines Oxidationsmittels ist.

11. Set zum Nachweis (1) von Biofilmen nach Anspruch 9, wobei die Entfärbungszusammensetzung (4) eine feste Phase eines Oxidationsmittels ist.

12. Set zum Nachweis (1) von Biofilmen nach Anspruch 10 oder 11, wobei das Oxidationsmittel ausgewählt ist aus der Gruppe bestehend aus Natronpercarbonat, Natriumhypochlorit, Wasserstoffperoxid, den Perboraten, Persulfaten oder auch Peroxiden oder deren Gemische und Derivate, wie beispielsweise Harnstoff-Percarbamat.

13. Set zum Nachweis (1) von Biofilmen nach einem der Ansprüche 8 bis 12, wobei die Verdünnungsphase 40 bis 50 Volumen-%, bevorzugter 45 Volumen-%, Ethanol, insbesondere reines Ethanol, in Bezug auf das Endvolumen der Verdünnungsphase, 8 bis 12 Volumen-%, insbesondere 10 Volumen-%, Essigsäure, insbesondere Eisessig, in Bezug auf das Endvolumen der Verdünnungsphase, und 40 bis 50 Volumen-%, bevorzugter 45 Volumen-%, Wasser in Bezug auf das Endvolumen der Verdünnungsphase umfasst.

14. Verwendung einer verdampfungsbereiten Lösung zum Färben von Biofilmen, bestehend aus einem Farbstoff, der Coomassie-Brillantblau ist, in Lösung in einer mit der Lebensmittelindustrie verträglichen Verdünnungsphase, umfassend 35 bis 55 Volumen-% Ethanol, 7 bis 13 Volumen-% Essigsäure und 35 bis 55 Volumen-% Wasser in Bezug auf das Endvolumen der Verdünnungsphase, und einer verdampfungsbereiten Reinigungslösung, die die Verdünnungsphase umfasst, auf einer Oberfläche, die möglicherweise durch einen Biofilm kontaminiert ist, wobei die Oberfläche eine offene Oberfläche ist, insbesondere in einer Lebensmittelindustrieanlage, um den Biofilm nachzuweisen und zu lokalisieren.

## Claims

1. Method for detecting biofilms, comprising the steps of:
- spraying a biofilm staining solution, consisting of a stain which is Coomassie Blue in solution in a dilution phase compatible with the agri-food industry, comprising from 35 to 55 vol% ethanol, from 7 to 13 vol% acetic acid and from 35 to 55 vol% water, with respect to the final volume of said dilution phase, onto a surface liable to be contaminated by a biofilm, said surface being an open surface in a plant,
- staining said biofilm with said staining solution for a predetermined period of less than 15 minutes,
- spraying a cleaning solution comprising said dilution phase,
- diluting said excess staining solution with said cleaning solution for a predetermined period of less than 15 minutes, and
- detecting and precisely locating said biofilm by observing residual areas stained with Coomassie Blue corresponding to the biofilms stained by said stain,
- said method for detecting biofilms being a method for detecting and precisely locating the biofilms in less than an hour and compatible with the agri-food industry.

2. Method for detecting biofilms according to claim 1, wherein one or more of the steps chosen from the group of the step of spraying said staining solution, spraying said cleaning solution and detecting said biofilm is preceded by a step of rinsing with water.

3. Method for detecting biofilms according to claim 1 or claim 2, further comprising a step of bleaching said residual areas stained with Coomassie Blue by applying a bleaching composition.

4. Method for detecting biofilms according to claim 3, wherein said bleaching step is carried out by applying a solid bleaching composition, in particular an oxidising agent in powder form.

5. Method for detecting biofilms according to claim 3, wherein said bleaching step is carried out by applying a liquid bleaching composition of an oxidising agent.

6. Method according to any one of claims 1 to 5, wherein said predetermined period ranges between 3 and 15 minutes, preferably between 4 and 10 minutes, and is generally 5 minutes.

7. Method according to any one of claims 1 to 6, wherein said open surface liable to be contaminated by a biofilm is a surface in a plant of the agri-food industry, water treatment, cooling circuit, animal health and nutrition.

8. Kit for detecting (1) and locating biofilms for implementation of the method according to any one of claims 1 to 7, comprising:
- a ready-to-spray biofilm staining solution (2) containing a stain in solution in a dilution phase compatible with the agri-food industry, comprising from 35 to 55 vol% ethanol, from 7 to 13 vol% acetic acid, and from 35 to 55 vol% water, with respect to the final volume of said dilution phase, wherein said stain is Coomassie Blue,
- a ready-to-spray cleaning spray (3) comprising said dilution phase,
- said kit for detecting and locating biofilms being compatible with the agri-food industry.

9. Kit for detecting (1) biofilms according to claim 8, further comprising a bleaching composition (4) compatible with the agri-food industry.

10. Kit for detecting (1) biofilms according to claim 9, wherein said bleaching composition (4) is an aqueous solution of an oxidising agent.

11. Kit for detecting (1) biofilms according to claim 9, wherein said bleaching composition (4) is a solid phase of an oxidising agent.

12. Kit for detecting (1) biofilms according to claim 10 or 11, said oxidising agent being chosen from the group of sodium percarbonate, sodium hypochlorite, oxygenated water, perborates, persulphates or else peroxides or mixtures and derivatives thereof such as, for example, urea percarbamate.

13. Kit for detecting (1) biofilms according to any one of claims 8 to 12, wherein said dilution phase comprises from 40 to 50 vol%, more preferentially 45 vol% ethanol, in particular absolute ethanol, with respect to the final volume of said dilution phase, from 8 to 12 vol%, more particularly 10 vol% acetic acid, in particular glacial acetic acid, with respect to the final volume of said dilution phase, and from 40 to 50 vol%, more preferentially 45 vol% water, with respect to the final volume of said dilution phase.

14. Use of a ready-to-spray biofilm staining solution, consisting of a stain which is Coomassie Blue in solution in a dilution phase compatible with the agri-food industry, comprising from 35 to 55 vol% ethanol, from 7 to 13 vol% acetic acid and from 35 to 55 vol% water, with respect to the final volume of said dilution phase, and a ready-to-spray cleaning solution comprising said dilution phase, on a surface liable to be contaminated by a biofilm, said surface being an open surface, in particular in a plant of the agri-food industry, in order to detect and locate said biofilm.
